(19)

Europäisches Patentamt
European Patent Office
Office européen des brevets

(11) **EP 2 952 212 A1**

(12) **EUROPEAN PATENT APPLICATION**

(43) Date of publication:
**09.12.2015 Bulletin 2015/50**

(51) Int Cl.:
***A61K 49/22*** (2006.01)

(21) Application number: **15170575.3**

(22) Date of filing: **03.06.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **03.06.2014 IT RM20140287**

(71) Applicant: **Paradossi, Gaio**
**00030 Labico (IT)**

(72) Inventor: **Paradossi, Gaio**
**00030 Labico (IT)**

(74) Representative: **Bosman, Cesare et al**
**Studio Torta S.p.A.**
**Via Viotti, 9**
**10121 Torino (IT)**

(54) **MICROVESICLES FOR PHOTOACOUSTIC APPLICATIONS AND METHOD FOR THE PREPARATION THEREOF**

(57)    A method for the preparation of microvesicles useful as contrast agent in photoacoustic applications; said method being characterised in that it comprises the steps of:
- preparing a graphene water suspension, during which a water solution of graphene and of a first surfactant, which comprises an aromatic structure suited to interact with graphene and reactive functional groups suited to perform a cross-linking step, is subject to the action of ultrasounds;
- emulsion, during which at least one graphene suspension produced in the previous step, a low boiling point perfluorocarbon, a second surfactant, and a biodegradable polymer functionalised with reactive functional groups suited to cause a cross-linking reaction of the biodegradable polymer with the first surfactant present in the graphene suspension are added to and emulsified in water;
- cross-linking, during which the reactive functional groups present in the biodegradable polymer and in the first surfactant cause a cross-linking process with the formation of microvesicles which include the perfluorocarbon.

**Description**

The present invention concerns microvesicles for photoacoustic applications and a method for the preparation thereof.

Here and below by microvesicles we mean hollow colloidal microparticles suited to retain inside them a liquid substance and which, once the liquid substance is evaporated, are able to retain the resulting gas and are reversibly transformed into microbubbles.

Here and below by low boiling point perfluorocarbon we mean a perfluorocarbon liquid with a boiling temperature equal to or lower than 60°C in ambient pressure condition.

The photoacoustic effect is a physical phenomenon that consists in the transformation of luminous energy into sound waves.

This effect has been exploited in photoacoustic spectroscopy for the detection of an acoustic signal through the non-radiative decay channel. Said information has been very important in the study of various materials, in particular semiconductors.

Recently the possibility of using the photoacoustic effect in medical diagnostics has been ascertained, exploiting the photoacoustic signal produced by the red blood cells.

The potential of microbubbles in the medical field, in both diagnostic and therapeutic terms, has been known for some time. In particular, microbubbles are advantageously used as contrast agents in ultrasound scanning.

Various microbubbles for ultrasound scanning are available on the market: air-filled denatured human serum albumin microbubbles, phospholipid and liposome-based microbubbles filled with gas such as SF6 and perfluorinated hydrocarbons.

Since the microbubbles are inserted into the human body, their component materials must be both biocompatible and, above all, biodegradable.

A further characteristic required of the microbubbles relates to their ability to remain intact during the entire period of their application. In said regard, in the medical field the need is felt to improve the stability of the microbubbles both in order to strengthen the efficiency of their application and also to be able to increase their field of application.

Usually, the microbubbles are formed of lipid chains held together by electrostatic bonds and consequently they have a particularly short lifetime. Said characteristic represents a significant limitation both in terms of efficiency and in terms of applicability of the microbubbles. In fact, the use of said microbubbles as contrast agents in ultrasound scanning imposes very limited analysis times on the personnel in charge.

The inventors of the present invention have produced microvesicles useful as contrast agents in photoacoustic applications, in particular in the sector of medical diagnostics. The microvesicles subject of the present invention have technical characteristics such as to guarantee them a high stability in the long term, reversible transformation into microbubbles when subject to the action of an appropriate luminous radiation and an amplification of the photoacoustic signal.

The subject of the present invention is a method for producing microvesicles, the essential characteristics of which are described in claim 1, and the preferred and/or auxiliary characteristics of which are described in the claims 2-9.

A further subject of the present invention are contrast agents for photoacoustic applications obtained with the microvesicles produced with the method subject of the invention.

Embodiment examples are described below for purely illustrative non-limiting purposes.

Materials: the polyethylene glycol 2,4,6-tris(1-phenylethyl)phenyl ether methacrylate) used as surfactant and the synthetic graphite powder with granulometry < 20 $\mu$m were purchased from Sigma Aldrich, Irgacure 2959 is a BASF product (Kaisten, CH) and the Epikuron 200 was purchased from Cargill (Hamburg, GE). The solvents were purchased from Carlo Erba (Milan, Italy). All the other chemicals were purchased from Sigma Aldrich (Milan, Italy). The Milli-Q water (18.2 M$\Omega$·cm) was produced with a PureLab deionizer by USF.

- Preparation of a graphene suspension -

A graphene suspension was prepared by adding graphite powder in water at a concentration of 20 mg/mL to 10 ml of a 0.625 mM solution of polyethylene glycol-2,4,6-tris(1-phenylethyl)phenyl ether methacrylate.

The concentration of surfactant must be just below the critical micelle concentration.

The mixture thus obtained, cooled in a water and ice bath, was subject to the action of a 320 W sonicator for ninety minutes.

The purpose of the sonication was to break the interactions between graphene layers (exfoliation) and help the surfactant to stabilise the exfoliated layers through the interaction between aromatic rings of the surfactant itself and the graphene. In particular, every 30 minutes the mixtures were stirred at 1500 rpm for 2 minutes in order to homogenise the graphite powder not yet exfoliated to obtain a greater exfoliation yield. The resulting dispersions were then centrifuged for 1 hour at 1300 rpm to remove the largest aggregates.

The concentration of 0.3 mg/ml of the graphite/surfactant system in water was measured by absorbance at 660 nm, using an extinction coefficient $\varepsilon = 1390$ mL mg$^{-1}$m$^{-1}$).

- Synthesis of dextran methacrylate -

5 g of dextran (Mw 35000-40000 g/mol), corresponding to $3.1 \times 10^{-2}$ moles of repeat units, were dispersed in 100 mL of dimethyl sulfoxide (DMSO) in a nitrogen atmosphere. 7.59 g of 4-(dimethyl amino)-pyridine, DMAP, ($6.2 \times 10^{-2}$ moles) were added and the mixture was left for 2 hours in the dark under stirring, followed by the addition of 2.2 g of glycidyl methacrylate ($1.6 \times 10^{-2}$ moles). The reaction was carried out for 48 hours at ambient temperature in the dark under vigorous stirring, and was interrupted by neutralising the DMAP with an equimolar quantity of HCl. The product was then purified by means of prolonged dialysis against water and kept as lyophilized powder.
The substitution with the methacrylate group was determined by means of 1H-NMR spectroscopy with a polymer concentration of 7 mg/mL in $D_2O$ using a Bruker Avance 300 MHz.
The degree of substitution of approximately 50% was determined as the ratio of the areas of the resonance peaks of the methacrylate protons (2 protons, 5.8 and 6.2 ppm) with respect to the area of the signal of the anomer peak at 5 ppm.

- Preparation of graphene-filled microvesicles -

The microvesicles with graphene were prepared by homogenising with an UltraTurrax T-25 IKA (Germany) at 15000 rpm for 5 minutes three different systems differing from one another according to the different concentration of graphene used. Each of the three systems is composed of 10 ml of a water suspension of graphene deriving from the one previously prepared and having a variable concentration as described below, and a mixture composed of 1.1% (weight/volume) of dextran methacrylate previously prepared, 0.005% (weight/volume) of Epikuron200, 0.002% of palmitic acid, 7% (weight/volume) of perfluoropentane and Irgacure 2959 at 0.3% (weight/weight) with respect to the polymer (all the concentrations refer to 10 mL of graphene suspension).
In particular, the following graphene solutions were used: Solution A: 10 ml of the graphene solution prepared above at a concentration of 3.0 mg/ml;
Solution B: 5 ml of the graphene solution prepared above at a concentration of 3.0 mg/ml + 5 ml water, obtaining 10 ml of a graphene solution at a graphene concentration of 1.5 mg/ml; Solution C: 1.7 ml of the graphene solution prepared above at a concentration of 3.0 mg/ml + 8.3 ml of water, obtaining 10 ml of a graphene solution at a graphene concentration of 0.5 mg/ml.
Each of the systems with different graphene concentration was irradiated with a UV lamp at 565 nm with an intensity of 7 mW/cm$^2$ in a water/ice bath for 20 minutes and left all night in the dark.
To summarise, the microvesicles with dextran methacrylate shell functionalised with graphene were prepared by high-speed homogenization of the components, followed by photo-copolymerization of the methacrylic groups of the surfactant which stabilises the graphene in water with the methacrylate groups of the lateral chains of the dextran methacrylate. The mixture was left to react all night and then washed with distilled water to obtain samples at different grapheme concentration. The method described led to the production of microvesicles with a mean diameter $4 \pm 1$ $\mu$m.
The species that did not react were removed by centrifugation at 1000 rpm for 4 minutes. The microvesicles were collected as pellets in the test tube and were then re-suspended in an appropriate volume of water and counted. The count of the microvesicles was conducted using the Neubauer chamber. The microvesicles were viewed with a Nikon inverted optical microscope with a 20x lens and the microphotographs acquired were processed with the ImageJ program.
The percentage of graphene filled was evaluated by subtracting the excess m, evaluated from the absorption at 660 nm, from the total mass $m_i$ according to the equation:

```
Graphene filled (%) = (mi-m)/mi x 100
```

Table I shows the results relative to the microvesicles produced from different quantities of graphene.

TABLE I

| | mg of Graphene used in suspension (10 ml) | % of graphene filled in the microvesicles | Number of microvesicles filled with graphene per ml | mg of Graphene filled (10 ml) |
|---|---|---|---|---|
| Suspension A | 3.0 | $50\pm5$ | $6\times10^8$ | 1.28 |
| Suspension B | 1.5 | $50\pm10$ | $2\times10^8$ | 0.6 |
| Suspension C | 0.5 | $40\pm7$ | $2\times10^8$ | 0.17 |

- Verification of stability of the microvesicles with graphene -

The microvesicles were marked with Rhodamine B isothiocyanate (RBITC) according to a known technique not described here. The marking with RBITC was conducted to prove the presence of polymer (dextran) on the shell of the microvesicles since the RBITC can react with the hydroxyls of the dextran chains.

The marked microvesicles were then subjected to the action of the ultrasounds by means of a generator (KTAC-4000, Sonidel) at a frequency of 1000 KHz. The action of the ultrasounds caused the evaporation of the perfluorocarbon with consequent transformation of the microvesicles into microbubbles. The water suspension of microbubbles produced was observed with confocal microscope.

The microbubbles were left to balance for 15 minutes to verify their stability. During the 15 minutes it was verified that the energy was dissipated in the water and the microbubbles returned to their original state of microvesicles maintaining their polymer wall intact.

- Photoacoustic applications of the microvesicles implemented with graphene. -

Graphene is a planar (2D) material with honeycomb structure and with particular electronic and optical properties. The outermost electrons have a delocalization similar to metals but confined to two dimensions. The graphene efficiently absorbs the electromagnetic radiation at the frequencies of the near infrared (NIR). Coupled with the microvesicles with polymer shell, the possibility can be exploited of transferring the energy accumulated by the graphene to the microvesicles in the form of mechanical energy which is dissipated by the microbubbles/microvesicles as vibrations due to the ultrasounds which are detected by an acoustic receiver. The advantages of using this system in photoacoustic diagnostics is the increase in the signal. Furthermore the particles (biocompatible) can be used also for the active transport of drugs, modifying the surface of the particles with molecules that recognise specific receptors/markers of tissues to be treated with drugs.

**Claims**

1. A method for the preparation of microvesicles useful as contrast agent in photoacoustic applications; said method being **characterised in that** it comprises:

   - a graphene water suspension preparation step, during which a water solution of graphene and of a first surfactant, which comprises an aromatic structure suited to interact with graphene and reactive functional groups suited to perform a cross-linking step, is subject to the action of ultrasounds so as to perform an exfoliation of graphene and encourage the interaction between the aromatic structure of the surfactant and the graphene;
   - an emulsion step, during which at least one graphene suspension produced in the previous step, a low boiling point perfluorocarbon, a second surfactant, and a biodegradable polymer with reactive functional groups suited to cause a cross-linking reaction of said biodegradable polymer with said first surfactant available in the graphene suspension are added to and emulsified in water;
   - a cross-linking step, during which the reactive functional groups available in the biodegradable polymer and in the first surfactant cause a cross-linking process with the formation of microvesicles including the perfluorocarbon.

2. A method according to claim 1, **characterised in that**, during the graphene water suspension preparation step, the concentration of the surfactant is lower than its critical micelle concentration.

3. A method according to claim 1, **characterised in that** said reactive functional groups are unsaturated alkyl groups and the cross-linking step comprises a radical activation operation.

4. A method according to claim 3, **characterised in that** said radical activation operation comprises a UV radiation.

5. A method according to any of the previous claims, **characterised in that** the biodegradable polymer has charges and **in that** the surfactant has a polar head with a charge that is opposite to the one of the biodegradable polymer.

6. A method according to any of the previous claims, **characterised in that**, during the emulsion step, the concentration of graphene in the suspended form ranges from 6 to 0.1 mg/ml, the biodegradable polymer is available at a concentration ranging from 0.3 to 3% (w/w), the perfluorocarbon is available at a concentration ranging from 4 to 7% (v/v), and the surfactant is available at a concentration ranging from 5 to 8% (w/w).

7. A method according to any of the previous claims, **characterised in that** the low boiling point perfluorocarbon is comprised in the group consisting of n-decafluoropentane, n-dodecafluoropentane, iso-dodecafluoropentane, cyclodecafluoropentane, or mixtures thereof.

8. A method according to any of the previous claims, **characterised in that** the biodegradable polymer is comprised in the group consisting of dextran, chitosan, hyaluronic acid, pullulan, agarose and alginic acid.

9. A method according to any of the previous claims, **characterised in that** said biodegradable polymer is polyethylene glycol-conjugated.

10. A contrast agent for photoacoustic analysis **characterised in that** it comprises microvesicles produced according to one of the previous claims.

Europäisches
Patentamt

European
Patent Office

Office européen
des brevets

## EUROPEAN SEARCH REPORT

Application Number

EP 15 17 0575

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 99/30620 A1 (IMARX PHARMACEUTICAL CORP [US]) 24 June 1999 (1999-06-24) * page 16, line 32 * * page 19, line 32 - page 21, line 6 * * examples 1,2 * | 1-10 | INV. A61K49/22 |
| A | GAURAV LALWANI ET AL: "Graphene-based contrast agents for photoacoustic and thermoacoustic tomography", PHOTOACOUSTICS, vol. 1, no. 3-4, 1 December 2013 (2013-12-01), pages 62-67, XP055164974, ISSN: 2213-5979, DOI: 10.1016/j.pacs.2013.10.001 * abstract * | 1-10 | |
| A | IT RM20 120 290 A1 (CAVALLI ROBERTA; PARADOSSI GAIO) 22 December 2013 (2013-12-22) * paragraphs [0007], [0011] - [0019] * * claims * | 1-10 | TECHNICAL FIELDS SEARCHED (IPC)  A61K |

The present search report has been drawn up for all claims

| Place of search | Date of completion of the search | Examiner |
|---|---|---|
| The Hague | 28 October 2015 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 15 17 0575

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2015

| Patent document cited in search report | Publication date | Patent family member(s) | Publication date |
|---|---|---|---|
| WO 9930620 A1 | 24-06-1999 | AU 1931899 A<br>DE 69831755 T2<br>EP 1039834 A1<br>US 6123923 A<br>WO 9930620 A1 | 05-07-1999<br>13-07-2006<br>04-10-2000<br>26-09-2000<br>24-06-1999 |
| IT RM20120290 A1 | 22-12-2013 | | |

EPO FORM P0459